(19) Europäisches Patentamt
European Patent Office
Office européen des brevets

(11) Publication number: **0 434 074 A2**

# (12) EUROPEAN PATENT APPLICATION

(21) Application number: **90124988.8**

(51) Int. Cl.5: **C07D 311/16**

(22) Date of filing: **20.12.90**

(30) Priority: **22.12.89 JP 334212/89**
**22.12.89 JP 334213/89**
**22.12.89 JP 334214/89**

(43) Date of publication of application:
**26.06.91 Bulletin 91/26**

(84) Designated Contracting States:
**AT BE CH DE DK ES FR GB GR IT LI LU NL SE**

(71) Applicant: **MITSUI PETROCHEMICAL INDUSTRIES, LTD.**
**2-5, Kasumigaseki 3-chome Chiyoda-ku**
**Tokyo 100(JP)**

(72) Inventor: **Fukuoka, Daisuke, c/o Mitsui Petrochem. Ind. Ltd.**
**1-2 Waki 6-chome, Waki-cho**
**Kuga-gun, Yamaguchi-ken(JP)**
Inventor: **Ishitoku, Takeshi, c/o Mitsui Petrochem. Ind. Ltd.**
**1-2 Waki 6-chome, Waki-cho**
**Kuga-gun, Yamaguchi-ken(JP)**
Inventor: **Hashimoto Isao, c/o Mitsui Petrochem. Ind. Ltd.**
**1-2 Waki 6-chome, Waki-cho**
**Kuga-gun, Yamaguchi-ken(JP)**
Inventor: **Kihara, Noriaki, c/o Mitsui Petrochem. Ind. Ltd.**
**1-2 Waki 6-chome, Waki-cho**
**Kuga-gun, Yamaguchi-ken(JP)**
Inventor: **Takesue, Mitsuyuki, c/o Mitsui Petrochem. Ind.**
**Ltd., 1-2 Waki 6-chome, Waki-cho**
**Kuga-gun, Yamaguchi-ken(JP)**

(74) Representative: **Hansen, Bernd, Dr. Dipl.-Chem. et al**
**Hoffmann, Eitle & Partner Patent- und Rechtsanwälte Arabellastrasse 4**
**W-8000 München 81(DE)**

## (54) Process for the production of 1-lower alkoxy-1-lower alkyl-3-substituted phenylurea, and intermediate thereof and process for the production of the intermediate.

(57) A 1-lower alkoxy-1-lower alkyl-3-[4-(2-lower alkoxy-2,3-dihydro-7-benzopyranyloxy)phenyl]-urea is prepared by a process which comprises alkylating a novel 1-hydroxy-3-[4-(2-lower alkoxy-2,3-dihydro-7-benzopyranyloxy)-phenyl]urea with a corresponding alkylating agent selected from the group consisting of a sulfuric acid ester and an alkyl halide in the presence of a basic compound. The 1-lower alkoxy-1-lower alkyl-3-[4-(2-lower alkoxy-2,3-dihydro-7-benzopyranyloxy)phenyl]urea has a herbicidal activity. New intermediates and processes for production thereof are also disclosed.

EP 0 434 074 A2

# PROCESS FOR THE PRODUCTION OF 1-LOWER ALKOXY-1-LOWER ALKYL-3-SUBSTITUTED PHENYLUREA AND INTERMEDIATE THEREOF AND PROCESS FOR THE PRODUCTION OF THE INTERMEDIATE

Detailed Description of the Invention

This invention relates to a process for the production of 1-lower alkoxy-1-lower alkyl-3-substituted phenylurea, and an intermediate thereof and a process for the production of the intermediate. More specifically, it relates to an industrially advantageous process for the production of 1-lower alkoxy-1-lower alkyl-3-substituted phenylurea useful as a herbicide, and an intermediate thereof and a process for the production of the intermediate.

Development of herbicides which cause no damage on intended crops and can control detrimental weeds by application of a small amount are still under way nowadays.

Japanese Laid-open Patent Publication No. 10779/1988 proposes a variety of urea derivatives useful as a herbicide and a process for the production thereof. That is, as a urea derivative which is relevant to the subject matter of this invention, the above Publication discloses the general formula (a)

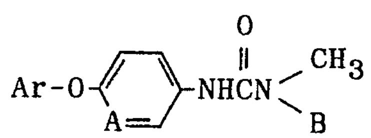

...(a)

wherein
Ar is a group of the formula

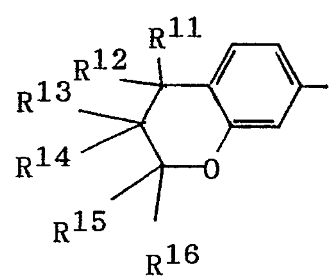

in which $R^{11}$ to $R^{15}$ may be the same or different and each is H, a lower alkyl or a lower alkoxy and $R^{16}$ is H, a lower alkyl, a lower alkoxy or OH,
A is -N= or

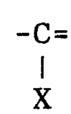

in which X is H, Cl, $NO_2$ or $CF_3$, and
B is H, $CH_3$ or $OCH_3$.

Further, the above Publication also discloses a process for the production thereof, which comprises reacting an aniline derivative (A = -CH=) or amino-pyridine derivative (A = -N=) of the formula (b)

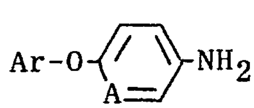

... (b)

wherein Ar and A are as defined in the formula (a), with methyl isocyanate, N,N-dimethyl carbamoyl chloride or N-methoxy-N-methyl carbamoyl chloride, and a process for the production thereof, which comprises reacting an isocyanate of the formula (c)

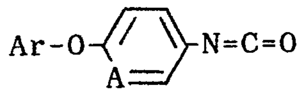

... (c)

wherein Ar and A are as defined in the formula (a), with an amine of the formula (d)

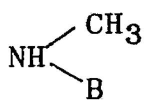

...(d)

wherein B is as defined in the formula (a).

Concerning the latter process above, said Publication discloses that the isocyanate of the formula (c) can be prepared from the corresponding Ar-OH compound according to a conventional method. However, no specific examples are described concerning the isocyanate of the formula (c).

It is an object of this invention to provide an industrially advantageous process for the production of 1-lower alkoxy-1-lower alkyl-3-substituted phenylurea useful as a herbicide.

It is another object of this invention to provide a process for the production of 1-lower alkoxy-1-lower alkyl-3-substituted phenylurea useful as a herbicide without using N-lower alkoxy-N-lower alkylamine such as N-methoxy-N-methylamine or N-methoxy-N-methyl carbamoyl chloride derived therefrom.

It is further another object of this invention to provide a process for industrially advantageous production of 1-lower alkoxy-1-lower alkyl-3-substituted phenylurea without using N-lower alkoxy-N-lower alkylamine which is a multistage reaction product and expensive or N-methoxy-N-methyl carbamoyl chloride which is greatly irritant to a mucous membrane and poor in storage stability.

Further, it is another object of this invention to provide a novel and useful intermediate for use in the above production processes of this invention, and an industrially advantageous process for the production thereof.

Other objects and advantages of this invention will be apparent from the following description.

The above objects and advantages of this invention are achieved, at first, by a process for the production of 1-lower alkoxy-1-lower alkyl-3-[4-(2-lower alkoxy-2,3-dihydro-7-benzopyranyloxy)phenyl]urea of the formula (I)

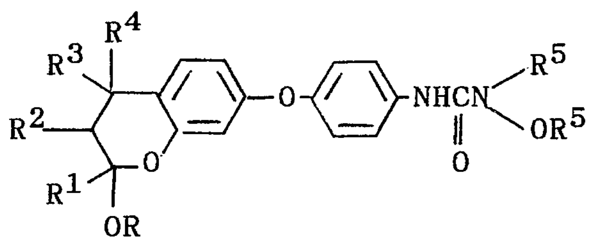

... (I)

wherein R is a lower alkyl group, $R^1$, $R^2$, $R^3$ and $R^4$ may be the same or diferent and each is a hydrogen atom or a lower alkyl group, and $R^5$ is a lower alkyl group,
which comprises alkylating 1-hydroxy-3-[4-(2-lower alkoxy-2,3-dihydro-7-benzopyranyloxy)phenyl]urea of the formula (II)

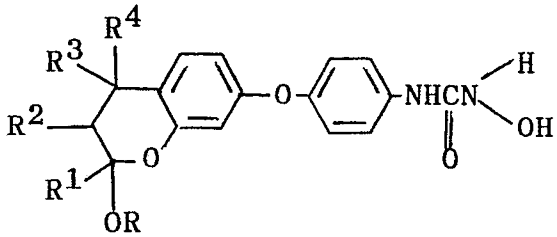

... (II)

wherein R, $R^1$, $R^2$, $R^3$ and $R^4$ are as defined above, with an alkylating agent selected from the group consisting of a sulfuric acid ester of the formula (III)-1

$$R^5\text{-}OSO_2O\text{-}R^5 \qquad ...(III)\text{-}1$$

wherein $R^5$ is as defined above, and an alkyl halide of the formula (III)-2

$$R^5\text{-}X \qquad ...(III)\text{-}2$$

wherein $R^5$ is as defined above and X is a halogen atom, in the presence of a basic compound.

The 1-lower alkoxy-1-lower alkyl-3-[4-(2-loweralkoxy-2,3-dihydro-7-benzopyranyloxy)phenyl]urea to be produced in this invention is represented by the above formula (I).

In the formula (I), R is a lower alkyl group, and the lower alkyl group may be linear or branched. The lower alkyl group preferably has 1 to 4 carbon atoms. Examples of such a lower alkyl group are methyl, ethyl, n-propyl, iso-propyl, n-butyl, sec-butyl, iso-butyl and tert-butyl groups. Of these groups, methyl and ethyl groups are preferred.

In the formula (I), $R^1$, $R^2$, $R^3$ and $R^4$ are the same or different, and each is a hydrogen atom or a lower alkyl group. This lower alkyl group may also be linear or branched, and preferably has 1 to 4 carbon atoms. Examples of such a lower alkyl group are the same as those specified above with regard to R. Methyl and ethyl groups are preferred as a lower alkyl group for each of $R^1$, $R^2$, $R^3$ and $R^4$, and a methyl group is particularly preferred.

Further, in the formula (I), $R^5$ is a lower alkyl group, and this lower alkyl group may also be linear or branched, and preferably has 1 to 4 carbon atoms. Examples of such a lower alkyl group are the same as those specified above with regard R. And, a methyl group is particularly preferred as a lower alkyl group for $R^5$.

Examples of the formula (I) are as follows.

(100) 1-methoxy-1-methyl-3-[4-(2-methoxy-2,3-dihydro-7-benzopyranyloxy)phenyl]urea,
(102) 1-methoxy-1-methyl-3-[4-(2-methoxy-2,4,4-trimethyl-2,3-dihydro-7-benzopyranyloxy)phenyl]-urea,
(104) 1-methoxy-1-methyl-3-[4-(2-ethoxy-2,4,4-trimethyl-2,3-dihydro-7-benzopyranyloxy)-phenyl]urea,
(106) 1-methoxy-1-methyl-3-[4-(2-n-propoxy-2,4,4-trimethyl-2,3-dihydro-7-benzopyranyloxy)phenyl]-urea,
(108) 1-methoxy-1-methyl-3-[4-(2-isopropoxy-2,4,4-trimethyl-2,3-dihydro-7-benzopyranyloxy)-phenyl]urea,
(110) 1-methoxy-1-methyl-3-[4-(2-ethoxy-2,3-dihydro-7-benzopyranyloxy)phenyl]urea,
(112) 1-methoxy-1-methyl-3-[4-(2-methoxy-4-methyl-2,3-dihydro-7-benzopyranyloxy)phenyl]urea,
(114) 1-methoxy-1-methyl-3-[4-(2-ethoxy-4-methyl-2,3-dihydro-7-benzopyranyloxy)phenyl]urea,
(116) 1-methoxy-1-methyl-3-[4-(2-methoxy-2-methyl-2,3-dihydro-7-benzopyranyloxy)phenyl]urea,
(118) 1-methoxy-1-methyl-3[4-(2-ethoxy-2 methyl-2,3-dihydro-7-benzopyranyloxy)phenyl]urea,
(120) 1-methoxy-1-methyl-3-[4-(2-iso-propoxy-2-methyl-2,3-dihydro-7-benzopyranyloxy)-phenyl]urea,
(122) 1-methoxy-1-methyl-3-[4-(2-methoxy-2-ethyl-2,3-dihydro-7-benzopyranyloxy)phenyl]urea,
(124) 1-methoxy-1-methyl-3-[4-(2-methoxy-2,4-dimethyl-2,3-dihydro-7-benzopyranyloxy)phenyl]urea,

(126) 1-methoxy-1-methyl-3-[4-(2-methoxy-2-ethyl-4-methyl-2,3-dihydro-7-benzopyranyloxy)-phenyl]urea, and

(128) 1-methoxy-1-methyl-3-[4-(2-methoxy-2,3-dimethyl-2,3-dihydro-7-benzopyranyloxy)phenyl]urea.

This invention provides a process for the production of the compound of the formula (I), which comprises alkylating the 1-hydroxy-3-[4-(2-lower alkoxy-2,3-dihydro-7-benzopyranyloxy)phenyl]urea of the formula (II) with an alkylating agent selected from the group consisting of the sulfuric acid ester of the formula (III)-1 and the alkyl halide of the formula (III)-2.

The compound of the formula (II) is a novel and constitutes part of this invention. In the formula (II), R and $R^1$ to $R^4$ are as defined in the formula (I).

Examples of the compound of the formula (II) are as follows.

(200) 1-hydroxy-3-[4-(2-methoxy-2,3-dihydro-7-benzo-pyranyloxy)phenyl]urea,
(202) 1-hydroxy-3-[4-(2-methoxy-2,4,4-trimethyl-2,3-dihydro-7-benzopyranyloxy)phenyl]urea,
(204) 1-hydroxy-3-[4-(2-ethoxy-2,4,4-trimethyl-2,3-dihydro-7-benzopyranyloxy)phenyl]urea,
(206) 1-hydroxy-3-[4-(2-n-propoxy-2,4,4-trimethyl-2,3-dihydro-7-benzopyranyloxy)phenyl]urea,
(208) 1-hydroxy-3-[4-(2-iso-propoxy-2,4,4-trimethyl-2,3-dihydro-7-benzopyranyloxy)-phenyl]urea,
(210) 1-hydroxy-3-[4-(2-ethoxy-2,3-dihydro-7-benzopyranyloxy)phenyl]urea,
(212) 1-hydroxy-3-[4-(2-methoxy-4-methyl-2,3-dihydro-7-benzopyranyloxy)phenyl]urea,
(214) 1-hydroxy-3-[4-(2-ethoxy-4-methyl-2,3-dihydro-7-benzopyranyloxy)phenyl]urea,
(216) 1-hydroxy-3-[4-(2-methoxy-2-methyl-2,3-dihydro-7-benzopyranyloxy)phenyl]urea,
(218) 1-hydroxy-3-[4-(2-ethoxy-2-methyl-2,3-dihydro-7-benzopyranyloxy)phenyl]urea,
(220) 1-hydroxy-3-[4-(2-iso-propoxy-2-methyl-2,3-dihydro-7-benzopyranyloxy)phenyl]urea,
(222) 1-hydroxy-3-[4-(2-methoxy-2-ethyl-2,3-dihydro-7-benzopyranyloxy)phenyl]urea,
(224) 1-hydroxy-3-[4-(2-methoxy-2,4-dimethyl-2,3-dihydro-7-benzopyranyloxy)phenyl]urea,
(226) 1-hydroxy-3-[4-(2-methoxy-2-ethyl-4-methyl-2,3-dihydro-7-benzopyranyloxy)phenyl]urea,
and
(228) 1-hydroxy-3-[4-(2-methoxy-2,3-dimethyl-2,3-dihydro-7-benzopyranyloxy)phenyl]urea.

The alkylating agent which reacts with the 1-hydroxy-3-substituted urea of the formula (II) to alklylate said hydroxy group is a sulfuric acid ester or an alkyl halide. The sulfuric acid ester is represented by the formula (III)-1, and the alkyl halide is represented by the formula (III)-2.

In the formulas (III)-1 and (III)-2, $R^5$ is as defined in the formula (I). In the formula (III)-2, X is a halogen atom, and it is preferably chlorine, bromine or iodine.

Examples of the sulfuric acid ester of the formula (III)-1 are dimethyl sulfate, diethyl sulfate, di-n-propyl sulfate, di-n-butyl sulfate, and the like.

Examples of the alkyl halide of the formula (III)-2 are methyl iodide, methyl bromide, ethyl iodide, ethyl bromide, n-propyl bromide, iso-propyl bromide, n-butyl bormide, and the like.

The above alkylating agents may be used alone or in combination. It is however preferred to use these individually.

The alkylation is carried out in the presence of a basic compound.

Examples of the basic compound are hydroxides, carbonates and alkoxides of alkali metals. Specific examples of the basic compound are sodium hydroxide, potassium hydroxide, sodium carbonate, potassium carbonate, sodium methoxide, sodium ethoxide, potassium methoxide and potassium ethoxide. Of these compounds, sodium hydroxide and potassium hydroxide are particularly preferred.

The amount of the basic compound for use is 2 to 10 moles, preferably 2.2 to 5 moles per mole of the 1-hydroxy-3-[4-(2-lower alkoxy-2,3-dihydro-7-benzopyranyloxy)phenyl]urea of the formula (II).

The amount of the alkylating agent of the formula (III)-1 or (III)-2 for use is 2 to 10 moles, preferably 2.2 to 5 moles per mole of the 1-hydroxy-3-[4-(2-lower alkoxy-2,3-dihydro-7-benzopyranyloxy)-phenyl]urea of the formula (II).

The alkylation may be carried out in the presence of a solvent. Examples of the solvent are preferably aromatic hydrocarbons such as benzene, toluene and xylene; halogenated hydrocarbons such as methylene chloride and 1,2-dichloroethane; ethers such as isopropyl ether and n-butyl ether; alcohols such as methanol, ethanol and iso-propanol; nitriles such as acetonitrile; and water. These solvents may be used alone or in combination. The amount of the solvent for use is 1 to 50 parts by weight, preferably 2 to 30 parts by weight based on 1 part by weight of the compound of the formula (I).

The alkylation is carried out at a temperature between -10 °C and 100 °C, preferably between 0 and 50 °C. The reaction time depends on an employed reaction temperature, and it is usually 0.5 to 40 hours, advantageously 1 to 20 hours.

The alkylation may be carried out in the presence of a phase transfer catalyst. Examples of the phase transfer catalyst are benzyltriethylammonium chloride and tetrabutylammonium iodide which are known as

such. The amount of the phase transfer catalyst for use is preferably 0.001 to 1 mol, more preferably 0.005 to 0.1 mol per mole of the compound of the formula (I). The use of the phase transfer catalyst improves the yield of the intended compound of the formula (I).

After the alkylation, the reaction mixture is subjected to a known post-treatment such as extraction, concentration, crystallization or chromatography, whereby the intended compound of the formula (I) can be obtained.

According to this invention, there is also provided a process for the production of the novel 1-hydroxy-3-[4-(2-lower alkoxy-2,3-dihydro-7-benzo-pyranyloxy)phenyl]urea of the formula (II), which comprises reacting 4-(2-lower alkoxy-2,3-dihydro-7-benzopyranyloxy)phenyl isocyanate of the formula (IV)

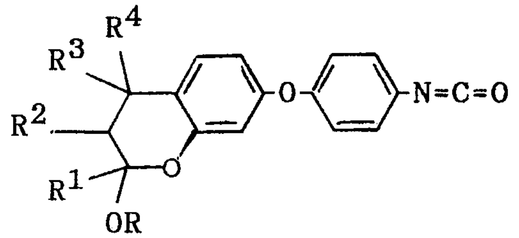 ... (IV)

wherein R, $R^1$, $R^2$, $R^3$ and $R^4$ are as defined above,
with a hydroxylamine.

The isocyanate of the formula (IV) is novel and constitutes part of this invention.

In the formula (IV), R, $R^1$, $R^2$, $R^3$ and $R^4$ are as defined in the formula (I).

Specific examples of the compound of the formula (IV) re as follows.

(400) 4-(2-methoxy-2,3-dihydro-7-benzopyranyloxy)-phenyl isocyanate,
(402) 4-(2-methoxy-2,4,4-trimethyl-2,3-dihydro-7-benzopyranyloxy)phenyl isocyanate,
(404) 4-(2-ethoxy-2,4,4-trimethyl-2,3-dihydro-7-benzopyranyloxy)phenyl isocyanate,
(406) 4-(2-n-propoxy-2,4,4-trimethyl-2,3-dihydro-7-benzopyranyloxy)phenyl isocyanate,
(408) 4-(2-iso-propoxy-2,4,4-trimethyl-2,3-dihydro-7-benzopyranyloxy)phenyl isocyanate,
(410) 4-(2-ethoxy-2,3-dihydro-7-benzopyranyloxy)phenyl isocyanate,
(412) 4-(2-methoxy-2-methyl-2,3-dihydro-7-benzopyranyloxy)phenyl isocyanate,
(414) 4-(2-ethoxy-4-methyl-2,3-dihydro-7-benzopyranyloxy)phenyl isocyanate,
(416) 4-(2-methoxy-2-methyl-2,3-dihydro-7-benzopyranyloxy)phenyl isocyanate,
(418) 4-(2-ethoxy-2-methyl-2,3-dihydro-7-benzo-pyranyloxy)phenyl isocyanate,
(420) 4-(2-iso-propoxy-2-methyl-2,3-dihydro-7-benzopyranyloxy)phenyl isocyanate,
(422) 4-(2-methoxy-2-ethyl-2,3-dihydro-7-benzopyranyloxy)phenyl isocyanate,
(424) 4-(2-methoxy-2,4-dimethyl-2,3-dihydro-7-benzopyranyloxy)phenyl isocyanate,
(426) 4-(2-methoxy-2-ethyl-4-methyl-2,3-dihydro-7-benzopyranyloxy)phenyl isocyanate, and
(428) 4-(2-methoxy-2,3-dimethyl-2,3-dihydro-7-benzopyranyloxy)phenyl isocyanate.

The hydroxylamine ($H_2NOH$) which is reacted with the 4-(2-lower alkoxy-2,3-dihydro-7-benzopyranyloxy)phenyl isocyanate of the formula (IV) can be obtained by reacting a hydroxylamine sulfate ($H_2NOH \cdot 1/2H_2SO_4$) or hydrochloride ($H_2NOH \cdot HCl$) with an inorganic base such as sodium hydroxide, potassium hydroxide, sodium carbonate, potassium carbonate, or the like. The amount of the base for use is usually 0.5 to 2.5 moles, preferably about 1 to 2.0 moles per mole of the hydroxylamine sulfate ($H_2NOH \cdot 1/2H_2SO_4$) or hydrochloride ($H_2NOH \cdot HCl$).

The amount of the hydroxylamine for use is preferably 0.5 to 10 moles, more preferably 0.5 to 5 moles per mole of the isocyanate of the formula (IV).

The reaction may be carried out in a solvent, preferably in a mixture system of water and an organic solvent. Examples of the organic solvent used for the reaction are aromatic hydrocarbons such as benzene, toluene and xylene; halogenated hydrocarbons such as carbon tetrachloride, 1,2-dichloroethane and chlorobenzene; and ethers such as iso-propyl ether and n-butyl ether.

The amount of the solvent for use is preferably 1 to 50 parts by weight, more preferably 2 to 30 parts by weight based on 1 part by weight of the isocyanate of the formula (IV).

The reaction is carried out at a temperature preferably between 0° C and 50° C, more preferably between 0° C and 25° C.

The reaction temperature depends on a reaction temperature. It is usually 0.1 to 20 hours, preferably 0.5 to 10 hours.

After the reaction, the reaction mixture is subjected to a known post-treatment such as extraction, concentration, crystallization or chromatography, whereby the intended 1-hydroxy-3[4-(2-lower alkoxy-2,3-dihydro-7-benzopyranyloxy)phenyl]urea of the formula (II) can be obtained.

According to this invention, there is further provided a process for the production of the novel isocyanate of the formula (IV), which comprises reacting a 4-(2-lower alkoxy-2,3-dihydro-7-benzopyranyloxy)aniline of the formula (V)

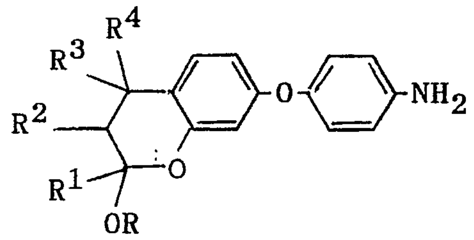

... (V)

wherein R, $R^1$, $R^2$, $R^3$ and $R^4$ are as defined above,
with phosgene in the presence of a basic compound.

In the formula (V), R, $R^1$, $R^2$, $R^3$ and $R^4$ are as defined in the formula (I).

Specific examples of the compound of the formula (V) are as follows.

(500) 4-(2-methoxy-2,3-dihydro-7-benzopyranyloxy)aniline.
(502) 4-(2-methoxy-2,4,4-trimethyl-2,3-dihydro-7-benzopyranyloxy)aniline,
(504) 4-(2-ethoxy-2,4,4-trimethyl-2,3-dihydro-7-benzopyranyloxy)aniline,
(506) 4-(2-n-propoxy-2,4,4-trimethyl-2,3-dihydro-7-benzopyranyloxy)aniline,
(508) 4-(2-iso-propoxy-2,4,4-trimethyl-2,3-dihydro-7-benzopyranyloxy)aniline,
(510) 4-(2-ethoxy-2,3-dihydro-7-benzopyranyloxy)aniline,
(512) 4-(2-methoxy-2-methyl-2,3-dihydro-7-benzopyranyloxy)aniline,
(514) 4-(2-ethoxy-4-methyl-2,3-dihydro-7-benzopyranyloxy)aniline,
(516) 4-(2-methoxy-2-methyl-2,3-dihydro-7-benzopyranyloxy)aniline,
(518) 4-(2-ethoxy-2-methyl-2,3-dihydro-7-benzopyranyloxy)aniline,
(520) 4-(2-iso-propoxy-2-methyl-2,3-dihydro-7-benzopyranyloxy)aniline,
(522) 4-(2-methoxy-2-ethyl-2,3-dihydro-7-benzopyranyloxy)aniline,
(524) 4-(2-methoxy-2,4-dimethyl-2,3-dihydro-7-benzopyranyloxy)aniline,
(526) 4-(2-methoxy-2-ethyl-4-methyl-2,3-dihydro-7-benzopyranyloxy)aniline, and
(528) 4-(2-methoxy-2,3-dimethyl-2,3-dihydro-7-benzopyranyloxy)aniline.

The 4-(2-lower alkoxy-2,3-dihydro-7-benzopyranyloxy)aniline of the formula (V) and phosgene ($COCl_2$) are reacted with each other in the presence of a basic compound.

Examples of the basic compound are preferably hydroxides, carbonates and bicarbonates of alkali metals, and N,N-dialkylanilines. Specific examples of the basic compound are inorganic bases such as sodium hydroxide, potassium hydroxide, sodium carbonate, potassium carbonate and sodium bicarbonate, and organic bases such as N,N-dimethylaniline and N,N-diethylaniline.

The amount of the basic compound for use is preferably 0.75 to 5 moles, more preferably 1 to 3 moles per mole of the compound of the formula (V).

The amount of the phosgene for use is preferably 1 to 2 moles, more preferably 1.05 to 1.3 moles per mole of the compound of the formula (V).

The reaction may be carried out in the presence of a solvent. Examples of the solvent are preferably aromatic hydrocarbons such as benzene, toluene and xylene; halogenated hydrocarbons such as carbon tetrachloride, 1,2-dichloroethane and chlorobenzene; ethers such as iso-propyl ether and n-butyl ether; esters such as methyl acetate and ethyl acetate; and water. These solvents may be used alone or in combination. The amount of the solvent for use is preferably 1 to 50 parts by weight, more preferably 2 to 30 parts by weight based on 1 part by weight of the compound of the formula (V).

The reaction temperature is preferably between -10 °C and 100 °C, more preferably between 0 °C and 25 °C.

The reaction time depends on a reaction temperature. It is usually 0.1 to 50 hours, preferably 0.25 to 20 hours.

After the reaction, the reaction mixture is subjected to a known post-treatment method such as concentration, distillation, etc., whereby the novel isocyanate compound of the formula (IV) can be obtained.

This invention will be more specifically explained by reference to Examples.

EXAMPLE 1

4-(2-Methoxy-2,4,4-trimethyl-2,3-dihydro-7-benzopyranyloxy)phenylisocyanate

A toluene solution (5 ml) of 0.594 g (6 mmol) of phosgene was cooled to 3° C with ice water. While the temperature inside was maintained at not more than 5° C, a toluene solution (9 ml) of 1.565 g (5 mmol) of 4-(2-methoxy-2,4,4-trimethyl-2,3-dihydro-7-benzopyranyloxy)aniline and 7.5 ml (7.5 mmol) of a 0.5 M $Na_2CO_3$ aqeuous solution were simultaneously added dropwise over 15 minutes. After the addition, the resultant mixture was stirred at said temperature further for 0.5 hour. A toluene layer was separated out, and was dried over anhydrous sodium sulfate. Toluene was distilled off under reduced pressure at room temperature to give 1.64 g of the subject product which was a light pink liquid (yield 97 %).

Mass spectrum m/z 339 (molecular ion peak)

$^1$H-NMR spectrum ($CDCl_3$ solution: ppm)
1.28(3H, s), 1.44(3H, s), 1.52(3H, s),
1.82(1H, d, J=14.1Hz), 2.04(1H, d, J=14.1Hz),
3.24( 3H, s), 6.46(1H, d, J=2.7Hz),
6.58(1H, dd, J=2.7, 9.0Hz),
7.01(3H, d, J=2.7Hz),
7.26(2H, d, J=2.7Hz).
IR spectrum (neat: $cm^{-1}$) 2275

EXAMPLES 2 and 3

Example 1 was repeated except that the amount of $Na_2CO_3$ was changed as shown in Table 1. Table 1 shows the results.

Table 1

| Example | Amount of base (mmol) | Yield (%) |
|---|---|---|
| 2 | 6.2 | 95.0 |
| 3 | 12.0 | 97.9 |

EXAMPLES 4-9

Example 1 was repeated except that the base and the amount and concentration thereof were changed as shown in Table 2. Table 2 shows the results.

Table 2

| Example | Base (mmol) | Base concentration (M) | Yield (%) |
|---|---|---|---|
| 4 | $K_2CO_3$ (6.0) | 1.36 | 94.4 |
| 5 | NaOH (12.0) | 1.0 | 94.9 |
| 6 | NaOH (12.0) | 2.0 | 94.7 |
| 7 | NaOH (12.0) | 4.0 | 94.1 |
| 8 | $NaHCO_3$ (12.0) | 0.6 | 82.5 |
| 9 | $Na_2CO_3$ (7.5) | 1.25 | 93.3 |

EXAMPLE 10

Example 1 was repeated except that 1,2-dichloroethane was used in place of toluene (yield 90 %).

COMPARATIVE EXAMPLE 1

A toluene solution (6.5 ml) of 1.9 g (19.2 mmol) of phosgene was cooled to 3° C with ice water. While the temperature inside was maintained at 3 to 4° C, 9.5 ml of a toluene solution of 2.0 g (6.4 mmol) of 4-(2-methoxy-2,4,4-trimethyl-2,3-dihydro-7-benzopyranyloxy)aniline was added dropwise over 0.5 hours. After the addition, the resultant mixture was stirred further for 0.5 hour at said temperature. Then, the reaction mixture was allowed to react for 0.5 hour while the temperature inside was maintained at 50 to 55° C. The reaction mixture was cooled and toluene was distilled off under reduced pressure (yield 15.7 %).

EXAMPLE 11

1-Hydroxy-3-[4-(2-methoxy-2,4,4-trimethyl-2,3-dihydro-7-benzopyranyloxy)phenyl]urea

A toluene solution (25 ml) of 297 g (30 mmol) of phosgene was cooled to 3° C with ice water. While the temperature inside was maintained at not more than 5° C, a toluene solution (45 ml) of 7.825 g (25 mmol) of 4-(2-methoxy-2,4,4-trimethyl- 2,3-dihydro-7-benzopyranyloxy)aniline and 15 ml of a 4M NaOH aqueous solution were simultaneously added dropwise over 15 minutes. After the addition, the resultant mixture was stirred further for 0.5 hours at said temperature. A toluene layer was separated out, and was washed with 30 ml of cold water to give a toluene solution of 4-(2-ethoxy-2,4,4-trimethyl-2,3-dihydro-7-benzopyranyloxy)-phenyl isocyanate.

Separately, 2.38 g (27.6 mmol) of a hydroxylamine sulfate ($H_2NOH \cdot 1/2H_2SO_4$) having a purity of 95 % was dissolved in 10 ml of water, and the resultant solution was cooled to 0° C. Added dropwise to this solution was an aqueous solution (16.2 ml) of 2.92 g (27.5 mmol) of $Na_2CO_3$ over 15 minutes while the temperature inside was maintained at 0° C.

The above toluene solution was added dropwise to the resultant aqueous solution at said temperature over 60 minutes. After the addition, the resultant reaction mixture was allowed to react further for 6 hours at room temperature.

Precipitated crystals were isolated by filtration, washed with water and then with hexane, and dried to give 7.45 g of the subject product as white crystals (yield 80 %).

Melting point: 107-108.5° C

Mass spectrum: m/z 372 (molecular ion peak)

$^1$H-NMR spectrum ($CD_3OD$ solution: ppm)

1.39(3H, s), 1.54(3H, s), 1.62(3H, s),
1.97(1H, d, J=14.1Hz), 2.17(1H, d, J=14.1Hz),
3.36(3H, s), 6.52(1H, d, J=2.7Hz),
6.70(1H, dd, J=2.7, 9.0Hz),
7.10(2H, d, J=9.0Hz), 7.42(1H, d, J=9.0Hz),
7.62(2H, d, J=9.0Hz).

EXAMPLES 12-14

Example 11 was repeated except that the amount of the hydroxylamine sulfate and the amount of $Na_2CO_3$ were changed as shown in Table 3. Table 3 shows the results.

Table 3

| Example | $H_2NOH \cdot 1/2H_2SO_4$ (mmol) | $Na_2CO_3$ (mmol) | Yield (%) |
|---|---|---|---|
| 12 | 37.6 | 37.5 | 83 |
| 13 | 50 | 50 | 93 |
| 14 | 100 | 100 | 93 |

EXAMPLES 15-17

Example 11 was repeated except that the base to neutralize the hydroxylamine sulfate was changed as shown in Table 4. Table 4 shows the results.

Table 4

| Example | Base | Yield (%) |
|---|---|---|
| 15 | NaOH | 78.5 |
| 16 | KOH | 80.3 |
| 17 | $K_2CO_3$ | 80.7 |

EXAMPLE 18

Example 11 was repeated except that the toluene was changed to 1,2-dichloroethane (Yield 83.5 %).

EXAMPLE 19

Example 11 was repeated except that the hydroxylamine sulfate was changed to a hydroxylamine

hydrochloride (Yield 79.4 %).

EXAMPLE 20

1-Methoxy-1-methyl-3-[4-(2-methoxy-2,4,4,-trimethyl-2,3-dihydro-7-benzopyranyloxy)-phenyl]urea

A 10N sodium hydroxide aqueous solution (0.55 ml, 5.5 mmol) was added dropwise to a mixture of 0.93 g (2.5 mmol) of 1-hydroxy-3-[4-(2-methoxy-2,4,4-trimethyl-2,3-dihydro-7-benzopyranyloxy)phenyl]urea, 0.73 g (5.5 mmol) of dimethyl sulfate and 5 ml of toluene with stirring and cooling with ice. After the addition, the resultant mixture was stirred further for 12 hours at room temperature. And, 2.5 ml of water was added to the resultant reaction mixture to separate the toluene layer. Then, toluene was distilled off under reduced pressure. The resultant concentrate was purified with silica gel column chromatography (development with hexane-ethyl acetate) to give 0.78 g of the subject product as white crystals (yield 78 %).

EXAMPLE 21

A 10N sodium hydroxide aqueous solution (0.44 ml, 4.4 mmol) was added dropwise to a mixture of 0.744 g (2 mmol) of 1-hydroxy-3-[4-(2-methoxy-2,4,4-trimethyl-2,3-dihydro-7-benzopyranyloxy)phenyl]urea, 4.6 mg (0.02 mmol) of benzyltriethylammonium chloride, 0.58 g (4.4 mmol) of dimethyl sulfate and 3.7 ml of toluene with stirring and cooling with ice. After the addition, the resultant mixture was stirred further for 6 hours at room temperature. Water (1.8 ml) was added to the resultant reaction mixture to separate the toluene layer, and then, toluene was distilled off under reduced pressure. The resultant concentrate was purified with a silica gel column chromatography (development with hexane-ethyl acetate) to give 0.64 g of an intended white crystal product (yield 80 %).

EXAMPLES 22-25

Example 21 was repeated except that the amounts of the dimethyl sulfate and the 10N sodium hydroxide aqueous solution were changed as shown in Table 5. Table 5 shows the results.

Table 5

| Example | Dimethyl sulfate (mmol) | 10N-NaOH (mmol) | Yield (%) |
|---|---|---|---|
| 22 | 4.8 | 0.48 | 81.3 |
| 23 | 6.0 | 0.48 | 82.6 |
| 24 | 8.0 | 0.48 | 83.7 |
| 25 | 4.8 | 0.44 | 82.1 |

EXAMPLE 26

Example 21 was repeated except that a 10N potassium hydroxide aqueous solution was used in place of the 10N sodium hydroxide aqueous solution (Yield 81 %).

EXAMPLE 27

A 2.5N sodium hydroxide aqueous solution (1.6 ml, 4.0 mmol) was added dropwise to a mixture of 0.372 g (1 mmol) of 1-hydroxy-3-[4-(2-methoxy-2,4,4-trimethyl-2,3-dihydro-7-benzopyranyloxy)phenyl]urea, 2.3 mg (0.01 mmol) of benzyltriethylammonium chloride, 0.53 g (4.0 mmol) of dimethyl sulfate and 3.7 ml of

toluene with stirring and cooling with ice. After the addition, the resultant mixture was stirred further for 6 hours at room temperature. Toluene (10 ml) and 1.8 ml of water were added to the resultant reaction mixture to separate the toluene layer, and then, toluene was distilled off under reduced pressure. The resultant concentrate was purified with a silica gel column chromatography (development with hexane-ethyl acetate) to give 0.31 g of an intended white crystal product (yield 77.5 %).

EXAMPLES 28-30

Example 21 was repeated except that the toluene was changed to other solvents as shown in Table 6. Table 6 shows the results.

Table 6

| Example | Solvent | Yield (%) |
|---|---|---|
| 28 | 1,2-dichloroethane | 83.7 |
| 29 | Acetonitrile | 75.6 |
| 30 | Methanol | 60.4 |

## Claims

1. A process for the production of 1-lower alkoxy-1-lower alkyl-3-[4-(2-lower alkoxy-2,3-dihydro-7-benzopyranyloxy)phenyl]urea of the formula (I)

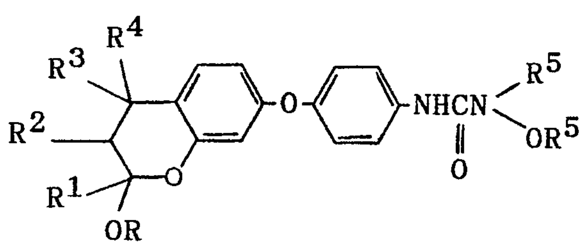

... (I)

wherein R is a lower alkyl group, $R^1$, $R^2$, $R^3$ and $R^4$ may be the same or diferent and each is a hydrogen atom or a lower alkyl group, and $R^5$ is a lower alkyl group,
which comprises alkylating 1-hydroxy-3-[4-(2-lower alkoxy-2,3-dihydro-7-benzopyranyloxy)phenyl]urea of the formula (II)

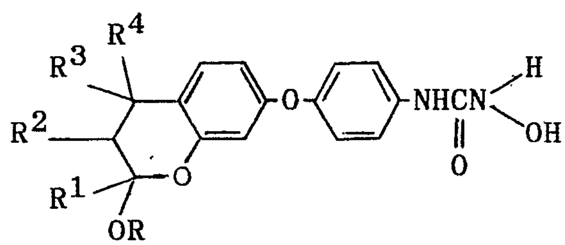

... (II)

wherein R, $R^1$, $R^2$, $R^3$ and $R^4$ are as defined above,
with an alkylating agent selected from the group consisting of a sulfuric acid ester of the formula (III)-1

$$R^5\text{-}OSO_2O\text{-}R^5 \qquad \ldots(III)\text{-}1$$

wherein $R^5$ is as defined above,
and an alkyl halide of the formula (III)-2

$$R^5\text{-}X \qquad \ldots(III)\text{-}2$$

wherein $R^5$ is as defined above and X is a halogen atom,
in the presence of a basic compound.

2. A process according to claim 1, wherein the basic compound is selected from the group consisting of hydroxides, carbonates and alkoxides of alkali metals.

3. A process according to claim 1, wherein the basic compound is used in an amount of 2 to 10 moles per mole of the compound of the formula (II).

4. A process according to claim 1, wherein the alkylating agent is used in an amount of 2 to 10 moles per mole of the compound of the formula (II).

5. A process according to claim 1, wherein the alkylation is carried out in the presence of a solvent.

6. A process according to claim 1, wherein the alkylation is carried out at a temperature between -10° C and 100° C.

7. 1-Hydroxy-3-[4-(2-lower alkoxy-2,3-dihydro-7-benzopyranyloxy)phenyl]urea represented by the formula (II).

8. A process for the production of the 1-hydroxy-3-[4-(2-lower alkoxy-2,3-dihydro-7-benzopyranyloxy)-phenyl]urea of the formula (II), which comprises reacting 4-(2-lower alkoxy-2,3-dihydro-7-benzopyranyloxy)phenyl isocyanate of the formula (IV)

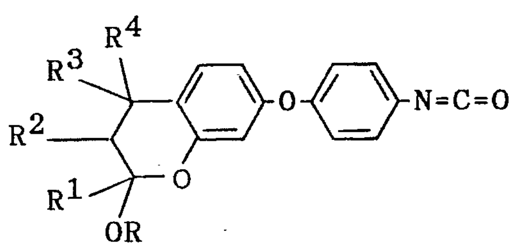

... (IV)

wherein R, $R^1$, $R^2$, $R^3$ and $R^4$ are as defined above,
with a hydroxylamine.

9. A process according to claim 8, wherein the hydroxylamine is used in an amount of 0.5 to 10 moles per mole of the isocyanate of the formula (IV).

10. A process according to claim 8, wherein the reaction is carried out in the presence of a solvent.

11. A process according to claim 8, wherein the reaction is carried out at a temperature between 0° C and 50° C.

**12.** 4-(2-Lower alkoxy-2,3-dihydro-7-benzopyranyloxy)phenyl isocyanate of the formula (IV).

**13.** A process for the production of the 4-(2-lower alkoxy-2,3-dihydro-7-benzopyranyloxy)phenyl isooyanate of the formula (IV), which comprises reacting a 4-(2-lower alkoxy-2,3-dihydro-7-benzopyranyloxy)aniline of the formula (V)

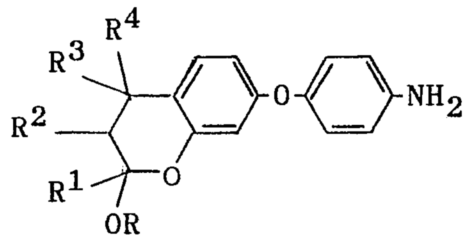

... (V)

wherein R, $R^1$, $R^2$, $R^3$ and $R^4$ are as defined above,
with phosgene in the presence of a basic compound.

**14.** A process according to claim 13, wherein the basic compound is selected from the group consisting of hydroxides, carbonates and bicarbonates of alkali metals and N,N-dialkylanilines.

**15.** A process according to claim 13, wherein the basic compound is used in an amount of 0.75 to 5 moles per mole of the compound of the formula (V).

**16.** A process according to claim 13, wherein the phosgene is used in an amount of 1 to 2 moles per mole of the compound of the formula (V).

**17.** A process according to claim 13, wherein the reaction is carried out in the presence of a solvent.

**18.** A process according to claim 13, the reaction is carried out at a temperature between -10°C and 100°C.